Europäisches Patentamt

(19) European Patent Office

Office européen des brevets

(11) Numéro de publication: **0 369 017**
**A1**

(12) **DEMANDE DE BREVET EUROPEEN PUBLI E EN APPLICATION DE L'article 158, paragraphe 3 de la CBE**

(21) Numéro de dépôt: **89901152.2**

(22) Date de dépôt: **26.05.88**

(86) Numéro de dépôt internationale :
**PCT/SU88/00132**

(87) Numéro de publication internationale :
**WO 89/11255 (30.11.89 89/28)**

(51) Int. Cl.⁵: **A61B 17/60**

(43) Date de publication de la demande:
**23.05.90 Bulletin 90/21**

(84) Etats contractants désignés:
**AT BE CH DE FR GB IT LI SE**

(71) Demandeur: **VSESOJUZNY KURGANSKY NAUCHNY TSENTR "VOSSTANOVITELNAYA TRAVMATOLOGIA I ORTOPEDIA"**
**ul. M.Ulyanovoi, 6**
**Kurgan, 640005(SU)**

(72) Inventeur: **ILIZAROV, Gavriil Abramovich**
**ul. Klimova, 41-38**
**Kurgan, 640020(SU)**
Inventeur: **BURLAKOV, Eduard Valentovich**
**ul. Klimova, 43-24**
**Kurgan, 640020(SU)**
Inventeur: **RUTS, Fridrikh Yakovlevich**
**ul. Klimova, 41-24**
**Kurgan, 640020(SU)**
Inventeur: **NEMKOV, Vitaly Anatolievich**
**ul. Komsomolskaya, 85-41**
**Kurgan, 640000(SU)**

(74) Mandataire: **Durand, Yves Armand Louis**
**CABINET WEINSTEIN 20, Avenue de**
**Friedland**
**F-75008 Paris(FR)**

(54) **APPAREIL A EXTENSION AUTOMATIQUE POUR OSTEOSYNTHESE.**

(57) L'appareil à extension automatique pour ostéo-synthèse suivant l'invention comporte deux centres d'appui (1) dont un appui (2) est muni de broches de fixation (3) y sont fixées, un actionneur (5) cinémati-quement réuni à des mécanismes par crochet et cliquet (7) qui comprennent une roue à crochet et un cliquet à ressort, selon l'invention, l'actionneur (5) et les mécanismes par crochet et cliquet (7) sont mon-tés sur un châssis (4) qui est placé entre les centres d'appui (1) et est relié à ces derniers, au moins l'un des centres d'appui (1) étant relié au châssis (4), avec possibilité de déplacement relatif, par le biais de tiges filetées d'extension (8) qui sont rigidement et coaxialement solidaires des roues à crochet et forment un système vis et écrou avec des écrous (9) qui se trouvent sur le centre d'appui (1) correspon-

dant.

*FIG.1*

"Appareil à extension automatique pour ostéosynthèse".

## Domaine de l'invention

L'invention se rapporte au génie médical et est utilisée dans le domaine de l'orthopédie et de la traumatologie pour le traitement de différents raccourcissements et défauts congénitaux ou acquis des os longs tubulaires, et se rapporte plus particulièrement aux appareils pour l'ostéosynthèse de fixation extérieure, et a trait plus précisément aux appareils à extension automatique pour l'ostéosynthèse.

## Etat de la technique

On connaît déjà un appareil pour la fixation externe des fragments osseux à la suite de fractures (US, A, 4502473). Cet appareil comporte un appui, réalisé en forme de tige prismatique portant deux paires de vis de fixation. L'appui porte un système pneumatique qui intéragit directement ou par le biais d'un levier avec un tirant pour actionner celui-ci. Le tirant est relié à l'une des paires de vis de fixation qui est installée de façon mobile par rapport à l'appui.

L'appareil susmentionné permet de stimuler la constitution du cal osseux au moyen d'une vibration qui se transmet dans la zone de la fracture osseuse.

Cependant ledit appareil connu ne peut pas être utilisé pour l'élongation des os longs tubulaires car il n'assure pas le déplacement relatif des paires de vis de fixation dans un sens donné et avec une cadence prédéterminée d'extension.

On connaît également un appareil de compression-extension (SU, A, 1122306) du type comprenant deux centres d'appui reliés à des écrous par des tiges filetées d'extension. L'un des centres d'appui supporte un actionneur en forme de ressort en spirale tendu, avec un mouvement d'horloge pour sa commande. L'actionneur est successivement relié aux écrous des tiges filetées d'extension à l'aide d'une transmission par chaîne.

L'appareil susmentionné ne permet cependant pas de changer le régime d'extension en cours de traitement. Dans le but de modifier l'amplitude du déplacement journalier du fragment osseux, il est nécessaire de démonter les paires de vis des systèmes vis et écrou (les tiges filetées avec leurs écrous respectifs qui sont installés dans l'appareil et en monter de nouvelles avec un filetage de pas différent. Cette manipulation n'est pas souhaitable puisque celle-ci peut entraîner un traumatisme des tissus biologiques ainsi que l'altération de leur processus de régénération. Par ailleurs, ledit appareil ne permet pas d'appliquer les forces requises pour réaliser une extension de l'os dans les conditions de charge d'appui du membre et de grandes valeurs des forces de résistance des tissus biologiques.

On connaît un autre appareil de compression-extension (SU, A, 84811) qui a deux centres d'appui qui sont reliés par des tiges filetées d'extension. Chacune des tiges filetées est munie de son propre actionneur comportant un électro-aimant, un mécanisme par crochet et cliquet ainsi qu'un réducteur avec un pignon-écrou qui est relié à la tige filetée d'extension. L'électro-aimant est branché à un bloc d'alimentation électrique.

Ledit appareil connu nécessite la présence d'au moins trois actionneurs installés sur l'appui, ce qui rend compliquée sa construction, augmente son encombrement et son poids, accroît sa consommation électrique, et oblige à recharger fréquemment des accumulateurs du bloc d'alimentation.

On connaît encore un autre appareil de compression-extension (SU, A, 1122308) du type comportant deux centres d'appui qui comprennent l'appui et des broches de fixation qui sont fixées au-dessus de celui-ci, l'actionneur étant en relation cinématique avec les mécanismes par crochet et cliquet qui sont eux-mêmes composés par une roue à crochet et un cliquet à ressort. Les appuis sont reliés entre eux au moyen de tiges filetées d'extension, vissées par l'une de ses extrémités dans les roues à crochet qui sont elles-mêmes montées à l'aide de paliers installés sur l'appui. L'actionneur est disposé sur le même appui et est relié aux mécanismes par crochet et cliquet au moyen d'un tirant formant câble qui est tendu à l'aide de galets de tension fixés sur l'appui.

Dans l'appareil connu et mentionné ci-dessus, l'actionneur ainsi que tous les mécanismes de sa liaison cinématique avec les tiges filetées d'extension qui sont installés sur l'appui sont encombrants et limitent la zone où peuvent se fixer les broches de fixation. Ceci oblige à courber les broches lors de leur fixation sur la partie libre de l'appui, ce qui provoque un déplacement du fragment osseux. D'autre part la disposition des centres d'appui sur les extrémités distale et les plus proches du segment osseux obliger à donner une longueur importante aux tiges filetées d'extension, ce qui diminue la rigidité de l'appareil et par conséquent réduit la rigidité de la fixation des fragments osseux, ce qui nuit à l'efficacité du traitement. La structure réalisée pour la jonction des tiges filetées d'extension avec les roues à crochet ainsi pour la jonction de la roue à

EP 0 369 017 A1

crochet avec l'appui, contribue à l'apparition de gauchissements dans le système vis et écrou dus à l'action de l'effort de tension du câble, ce qui entraîne des coincements du filet et donc oblige à augmenter la puissance de l'actionneur.

Exposé de l'invention

Le problème posé dans la présente invention consiste à mettre au point un appareil d'extension automatique pour ostéosynthèse qui, avec un actionneur de puissance minimale assurerait l'effort requis d'extension des fragments osseux avec une rigidité suffisante des fixation dans les centres d'appui, et avec la possibilité de fixer les broches de fixation dans une position prédéterminée dans les courbes ce qui entraîne comme on le sait le déplacement des fragments osseux.

Ce problème est résolu grâce à l'appareil à extension automatique pour ostéosynthèse, du type comportant deux centres d'appui qui comprennent un appui et des broches de fixation qui y sont fixées, ainsi qu'un actionneur cinématiquement lié à des mécanismes par crochet et cliquet qui comprennent la roue à crochet et le cliquet à ressort, et dans lequel, selon l'invention, l'actionneur et les mécanismes à crochet et cliquet sont montés sur un châssis qui est disposé entre les centres d'appui et y est relié, mais de façon à ce qu'au moins l'un des centres d'appui soit relié au châssis avec possibilité de déplacement relatif par le biais des tiges filetées d'extension qui sont rigidement et coaxialement reliées aux roues à crochet, en formant un système vis et écrou avec les écrous qui sont fixés sur le centre d'appui correspondant.

Avec une telle réalisation de l'appareil et quand un châssis est placé entre les deux centres d'appui il est possible de réduire la longueur des tiges filetées d'extension. Il en résulte un accroissement de la rigidité de l'appareil et donc de la fixation des fragments osseux, ce qui contribue à l'amélioration du processus de régénération. L'installation de l'actionneur et de ses blocs de liaison cinématique avec les tiges filetées d'extension sur le châssis de l'appareil libère l'emplacement sur l'appui prévu pour la fixation des broches en position requise sans avoir à les courber, évitant ainsi la possibilité de déplacement des fragments osseux par rapport à leur position correcte. En outre, grâce à la jonction rigide des tiges filetées d'extension avec les roues à crochet et grâce à l'emplacement des écrous sur le centre d'appui, on élimine tout risque de gauchissement du système vis et écrou sous l'influence d'une force latérale transmise par le biais des mécanismes à crochet et cliquet de l'actionneur. Une telle structure permet de réduire la puissance de l'actionneur, sa consommation électrique et son encombrement, et aussi de réduire la quantité de recharges de la batterie d'accumulateurs qui alimente l'actionneur.

Il est avantageux de réaliser l'appareil avec un actionneur qui comprend un moteur électrique et un réducteur, sur l'arbre duquel est rigidement fixée une came en forme de cylindre placé excentriquement, qui coopère avec un mécanisme de transformation du mouvement de rotation en mouvement alternatif, qui est relié par les cliquets à ressort des mécanismes par crochet et cliquet. Ces cliquets sont installés sur des bagues qui ont la possibilité de rotation autour d'un axe statiquement fixé sur le châssis, la bague étant installée coaxialement par rapport à la roue à crochet et avec possibilité de rotation relativement à cette dernière.

Une telle réalisation de l'appareil selon l'invention permet d'utiliser l'actionneur le plus économique, c'est-à-dire celui dont la liaison cinématique avec les cliquets des mécanismes à crochet et cliquet est la plus simple, donc ayant la structure la plus compacte et excluant la transmission des forces latérales au système vis et écrou.

L'appareil selon l'invention peut avoir un châssis en forme de boîtier fermé, et le mécanisme de conversion du mouvement rotatif en mouvement alternatif peut avoir la forme d'un palier dont la cage est fixée sur la came et qui s'accouple par paire avec les bagues au moyen d'un tirant.

Une telle réalisation de l'appareil selon l'invention permet d'augmenter le rendement du mécanisme de transformation du mouvement rotatif en mouvement alternatif et d'occuper un encombrement minimum en permettant ainsi de disposer l'actionneur sur le rebord extérieur du châssis, ce qui donne à l'appareil en lui-même un encombrement minimum.

Suivant une des variantes de réalisation de l'appareil selon l'invention, le châssis a un rebord extérieur ouvert et le mécanisme de conversion du mouvement rotatif en mouvement alternatif a la forme d'un fléau dont l'un des embouts comportent une fourche qui enserre la came alors que l'autre embout est placé dans une rainure réalisée à la périphérie de l'une des bagues, celle-ci étant successivement fixée et articulée aux autres bagues par l'intermédiaire de tirants.

Une telle réalisation de l'appareil selon l'invention permet d'utiliser le rebord extérieur ouvert des appuis que l'on place à proximité des articulations pour permettre les mouvements dans ces articulations, ainsi que les parties les plus proches du fémur et de l'humerus.

Suivant une autre variante de réalisation de l'appareil selon l'invention, le châssis est rigidement relié à l'un des centres d'appui, alors que les tiges filetées de l'extension sont placées de l'autre côté du plan du châssis, la roue à crochet étant disposée sur l'axe susmentionné avec possibilité de rotation rigidement fixé sur le châssis.

Une telle réalisation de l'appareil simplifie sa construction et permet d'effectuer la jonction de l'appui avec le châssis indépendamment l'un de l'autre à l'emplacement désiré, en pouvant ainsi fixer des broches de fixation sur les appuis à n'importe quel endroit.

Suivant encore une autre variante de réalisation de l'invention, les axes susmentionnés sont creux et les tiges filetées d'extension sont situées sur les différents côtés des roues à crochet, les tiges filetées d'extension qui se situent sur l'un des côtés des roues à crochet et sont installées avec possibilité de rotation à l'intérieur des axes creux.

Ceci rend possible le déplacement des centres d'appui par rapport au châssis de l'appareil.

Suivant l'une des variantes de réalisation de l'appareil selon l'invention, les tiges filetées d'extension qui sont situées de différents côtés des roues à crochet comportent des filets de sens opposés.

Ceci permet d'effectuer le déplacement des centres d'appui l'un par rapport à l'autre dans des sens différents relativement au châssis et par cela même d'accroître la vitesse d'extension.

Suivant une autre variante de réalisation de l'invention, les filets des tiges d'extension qui sont placés sur les différents côtés des roues à crochet comportent un pas différent.

Ceci permet de réaliser le déplacement des centres d'appui l'un par rapport à l'autre. D'autre part, en choisissant des pas différents on peut régler la cadence de déplacement relatif des centres d'appui avec un fractionnement moindre.

Il est avantageux de réaliser les tiges filetées d'extension situées des différents côtés des roues à crochet et avec des filets de même sens mais de pas différent.

Une telle exécution de l'appareil selon l'invention permet d'obtenir un déplacement relatif des centres d'appui à une cadence minimale qui correspond à la valeur de la différence des pas choisis pour un tour des tiges filetées d'extension.

Suivant l'une des variantes de réalisation de l'invention, le châssis comporte les broches de fixation.

Ceci permet, suite à l'ostéomie à deux niveaux d'un segment du membre d'effectuer une double extension, ce qui dans les conditions de cadence optimale d'extension permet de réduire de moitié la durée du traitement.

L'appareil selon l'invention peut également comporter des écrous en forme de tubes pour le système vis et écrou l'un des embouts étant relié au centre d'appui, alors que la tige filetée d'extension coopère avec un filetage interne réalisé à l'autre embout du tube.

Ceci permet d'éviter que les embouts des tiges filetées d'extension sortent de l'appui et qui, lors de l'installation des appuis près des articulations, limite leurs mouvements et de plus réduit la possibilité de réaliser simultanément l'ostéosynthèse par la pose d'appareils sur les parties adjacentes du membre.

Il est de plus avantageux de réaliser la jonction du tube avec le centre d'appui en forme d'articulation à rotule.

Grâce à une telle jonction, l'appareil selon l'invention rend possible, dans le cas où l'emplacement n'est pas parallèle aux appuis, d'exclure tout risque de coincement du système vis et écrou et ainsi de réduire la puissance et la consommation en énergie de l'actionneur.

Suivant l'une des variantes de réalisation de l'appareil selon l'invention, les éléments de l'articulation à rotule sont liés entre eux par l'intermédiaire d'un ressort.

Un mode de réalisation de l'articulation à rotule permet d'obtenir une amplitude suffisante du mouvement dans ladite articulation tout en maintenant un petit encombrement et une structure simple.

Suivant l'une des variantes de réalisation de l'invention, les roues à crochet sont exécutées avec des dentures de sens différents, les filets des tiges d'extension étant rigidement liés aux roues à crochet de sens correspondant.

Ceci permet, grâce aux différents sens des dentures des roues à crochet, d'utiliser le mouvement direct et inverse des tirants pour la rotation alternative des tiges filetées d'extension ce qui réduit encore la charge appliquée à l'actionneur.

Brève description des figures

L'invention sera mieux comprise à la lumière des exemples de réalisation non limitatifs qui suivent en référence aux dessins schématiques annexés dans lesquels :

La figure 1 représente schématiquement la vue générale de l'appareil selon l'invention.

La figure 2 représente schématiquement une partie du châssis de l'appareil, représenté dans la figure 1, avec l'actionneur et le mécanisme par crochet et cliquet.

La figure 3 représente schématiquement la vue générale d'une variante de réalisation de l'appareil selon l'invention.

La figure 4 représente une partie du châssis de la variante de l'appareil représentée dans la figure 3, avec l'actionneur et les mécanismes par crochet et cliquet.

La figure 5 représente l'articulation à rotule de la variante de l'appareil représentée dans la figure 3.

La figure 6 représente le schéma cinématique de l'appareil représenté dans la figure 1.

La figure 7 représente le schéma cinématique de

EP 0 369 017 A1

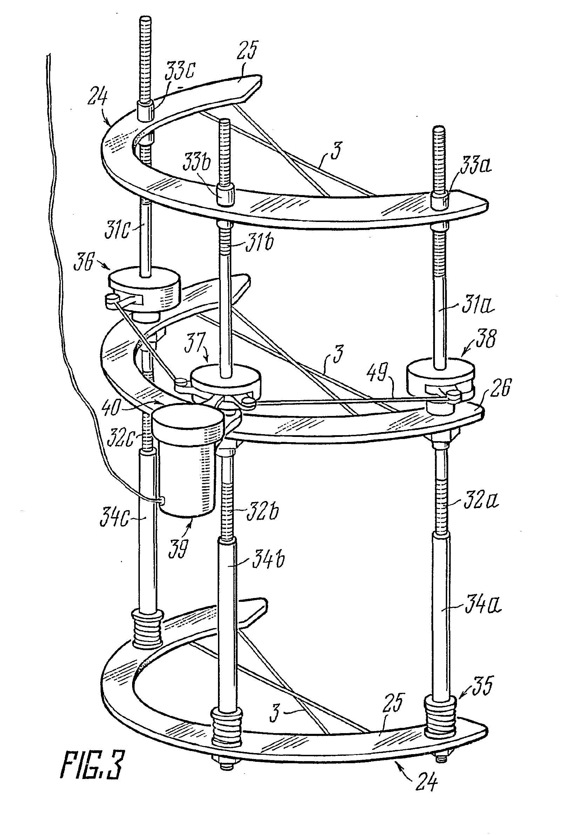

l'appareil représenté dans la figure 3.

## Description des meilleures variantes de réalisation

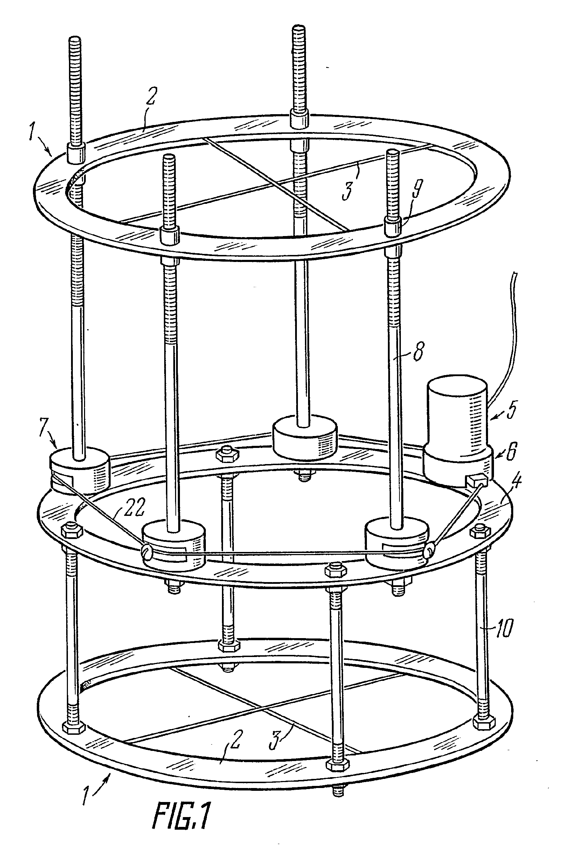

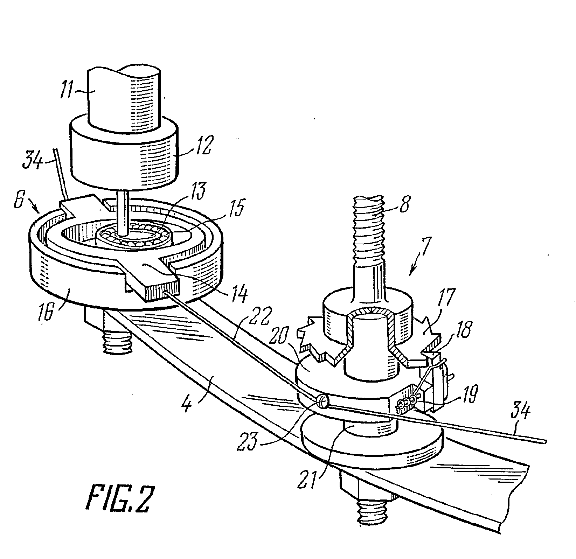

L'appareil pour ostéosynthèse à extension automatique selon l'invention comporte deux centres d'appui 1 (figure 1) comprenant des appuis 2 en forme de bague et d'arcs comportant des broches de fixation 3. Les éléments de fixation des broches ne sont pas représentés sur les figures. Entre les centres d'appui 1 est placé un châssis 4. Le châssis 4 comporte un actionneur 5 avec un mécanisme 6 de conversion du mouvement rotatif en mouvement alternatif ainsi que des mécanismes par crochet et cliquet 7. L'un des centres d'appui 1 (en haut sur le dessin) est solidaire du châssis 4 par le biais de tiges filetées d'extension 8 vissées sur des écrous 9 qui sont eux-mêmes fixés audit centre d'appui 1. L'autre centre d'appui 1 (en bas dans le dessin) est rigidement lié au châssis 4 au moyen de tiges 10. L'actionneur 5 comprend un moteur électrique 11 (figure 2), un réducteur 12 et une came 13. Le moteur électrique 11 est connecté par l'intermédiaire d'un circuit électrique à une source d'alimentation et à un bloc électronique de commande (non représentée dans les dessins). La came 13 a la forme d'un cylindre et est fixée excentriquement sur l'arbre du réducteur 12. Le mécanisme 6 de conversion du mouvement rotatif et alternatif comporte un boîtier 14 qui entoure la came 13 ainsi qu'un palier 15 y ajusté. Le boîtier 14 est installé dans un corps 16 rigidement fixé au châssis 4. Les mécanismes à crochet et cliquet 7 comprennent une roue à crochet 17 rigidement reliée à la tige filetée d'extension 8 ainsi qu'un cliquet 18 à ressort et un ressort 19. Le cliquet 18 est placé sur une bague 20. La roue à crochet 17 et la bague 20 sont installées, avec possibilité de rotation, sur un axe 21 qui est immobilement fixé au châssis 4. Le boîtier 14 est successivement relié aux bagues 20 par le biais d'un tirant souple 22 qui est fixé sur les bagues 20 à l'aide des vis 24.

Une telle réalisation de l'appareil selon l'invention permet d'effectuer automatiquement et avec une petite discontinuité de la sélection requise du nombre de dents des roues à crochet et du pas de filet des tiges d'extension, lors du déplacement des centres d'appui l'un par rapport à l'autre.

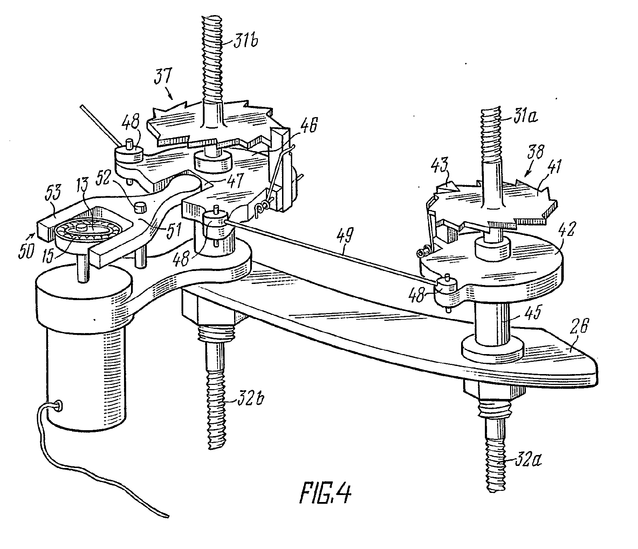

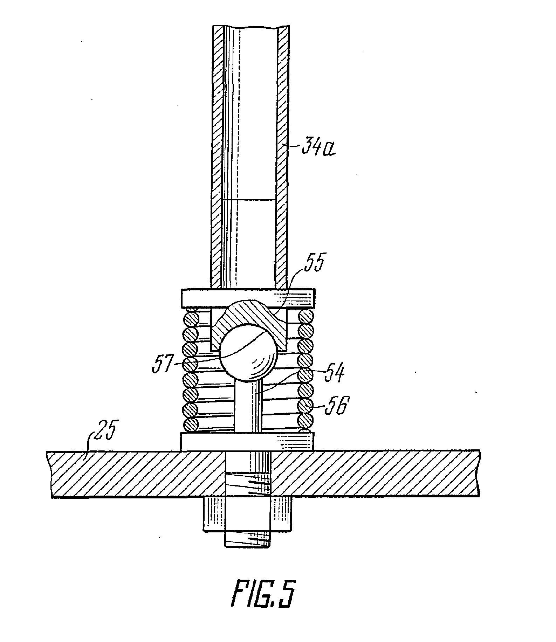

Suivant une autre variante de réalisation de l'appareil pour ostéosynthèse à extension automatique selon l'invention comporte deux centres d'appui 24 (figure 3), qui comprennent un appui 25 de support des broches de fixation 3. Entre les centres d'appui 24 est placé un châssis 26. Les appuis 25 et le châssis 26 ont un rebord extérieur ouvert en forme de fer à cheval. Sur le châssis 26 sont fixées les broches de fixation 3. Les centres d'appui 24 sont reliés au châssis au moyen de tiges filetées d'extension 31a, 31b, 31c et de tiges d'extension 32a, 32b, 32c, vissées dans des écrous 33a, 33b, 33c et 34a, 34b, 34c respectivement. Les écrous 33a, 33b, 33c sont fixés à l'appui supérieur (d'après le dessin), alors que les écrous 34a, 34b, 34c sont fixés à l'appui 25 inférieur (dans le dessin). Les tiges d'extension 31a, 31b, 31c sont exécutées avec un filet à gauche. Les écrous 34a, 34b, 34c sont réalisés en forme de tube et se fixent sur le centre d'appui par l'intermédiaire d'une articulation à rotule 35. Le châssis 26 comporte des mécanismes à crochet et cliquet 36, 37, 38 ainsi qu'un actionneur 39 muni d'un mécanisme 40 de conversion du mouvement rotatif en mouvement alternatif. Les mécanismes par crochet et cliquet 36 et 37 ont le même sens de rotation durant leur fonctionnement. Le mécanisme à crochet et cliquet 38 tourne en sens inverse durant son fonctionnement et comprend une roue à crochet 41 (figure 4) rigidement reliée aux tiges filetées d'extension 31a, 32a, ainsi qu'une bague 42 avec un cliquet à ressort 43. La tige d'extension 32a est disposée avec possibilité de rotation dans un axe creux 45, et la bague 42 est disposée, avec possibilité de rotation sur ledit axe creux 45. L'axe creux 45 est rigidement fixé sur le châssis 26. Les autres mécanismes à crochet et cliquet ont la même structure. Mais contrairement aux autres bagues, une bague 46 du mécanisme à crochet et cliquet 37 comporte une rainure 47 et deux charnières 48 pour la jonction successive des bagues des mécanismes à crochet et cliquet au moyen de tirants rigides 49. Un mécanisme 50 pour la conversion du mouvement rotatif en mouvement alternatif comprend un fléau 51 installé sur un axe 52. L'un des embouts du fléau 51 comporte une fourche 53 qui entoure la came 13 et le palier 15. L'autre embout du fléau est placé dans la rainure 47 de la bague 46. L'articulation à rotule 35 comporte un montant sphérique 54 (figure 5) et un pivot 55 qui sont reliés entre eux par un ressort 56. Le pivot 55 est exécuté avec une surface sphérique 57.

Une telle réalisation de l'appareil selon l'invention permet d'effectuer le déplacement des centres d'appui l'un par rapport à l'autre dans des sens différents par rapport au châssis qui est immobile. Ceci permet d'effectuer l'extension à deux niveaux d'un même segment du membre.

Une autre variante de réalisation de l'appareil pour ostéosynthèse à extension automatique selon l'invention est analogue à la précédente réalisation à cela près que les filets des tiges d'extension 31a, 31b, 31c et creux des tiges d'extension 32a, 32b, 32c ont un pas différent.

Cela permet, à une sélection requise des pas du filet, de réaliser de différentes cadences d'extension aux différents niveaux, en fonction des diverses conditions de régénération des tissus biologiques.

Suivant une variante de réalisation de l'appareil selon l'invention, dans le cas où le châssis 26 ne comporte pas de broches de fixation, la variante peut être exécutée de façon analogue à la précédente à cela près que les filets des tiges filetées d'extension 31a, 31b, 31c et des tiges d'extension 32a, 32b, 32c sont réalisés dans le même sens mais avec un pas différent.

Une telle réalisation de l'appareil permet d'effectuer le déplacement relatif des centres d'appui 24 sur la grandeur de la différence des pas des filets pour un tour de leur rotation.

L'appareil pour ostéosynthèse à extension selon l'invention, destiné à l'allongement des membres fonctionne comme suit.

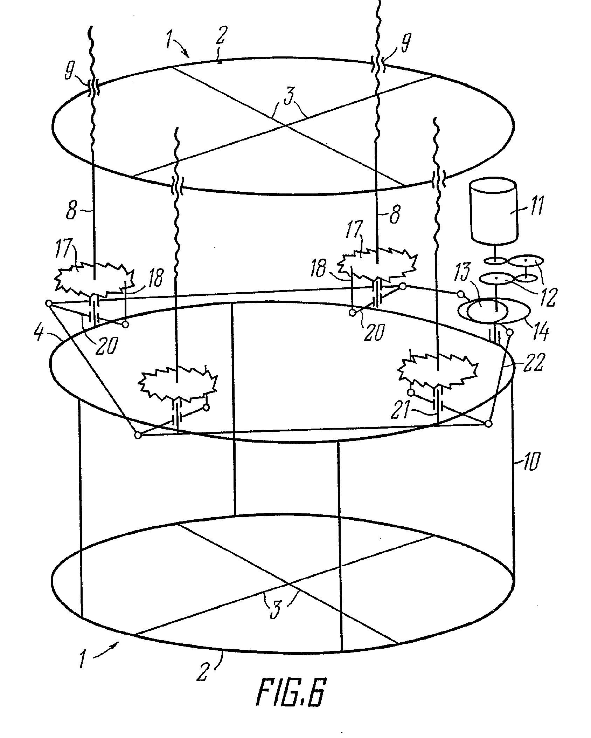

Avant d'effectuer l'ostéotomie, on monte l'appareil selon l'invention de façon que la fixation des parties les plus proches et distales de l'os soit effectuée par les broches 3 (figure 6) fixées dans les appuis 2. Après un certain délai après l'ostéotomie, on branche le moteur électrique 11 à la source d'alimentation à l'aide du bloc de commande électronique. Il en résulte un enclenchement périodique du moteur électrique 11. La rotation de l'arbre du moteur 11 est transmise par le biais du réducteur 12 jusqu'à la came 13 qui assure le mouvement alternatif de la boîte 14. Par le biais du tirant souple 22 la boîte 14 transmet les mouvements de roulement aux bagues 20 et aux cliquets 18 qui y sont installés. Les cliquets 18 transmettent le roulement alternatif aux roues à crochet 17 avec les tiges d'extension 8. Les tiges filetées d'extension 8 se dévissant des écrous 9 et provoquent le déplacement relatif de l'un des centres d'appui 1 par rapport à l'autre qui est rigidement fixé sur le châssis 4. Les fragments osseux fixés aux centres d'appui 1 se déplacent à la cadence requise, déterminée par le bloc de commande électronique.

La variante de l'appareil pour ostéosynthèse à extension automatique selon l'invention, destinée à l'allongement des membres à deux niveaux, fonctionne comme suit.

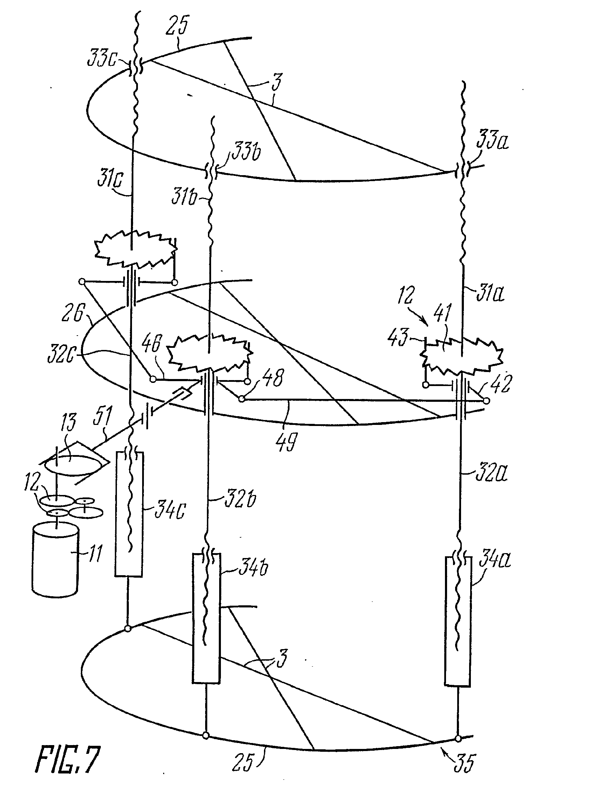

On met en place sur le membre, l'appareil assemblé, on fait passer les broches de fixation 3 à travers les parties la plus proche, distale et médiane de l'os (figure 7) suite à quoi on fixe respectivement ces dernières dans les appuis 25 et le châssis 26. On effectue alors deux ostéotomies à deux niveaux, distalement et au plus proche par rapport aux broches fixées sur le châssis. A l'issue d'un certain temps, on branche le moteur électrique 11 à la source d'alimentation avec le bloc de commande électronique. En accord un programme prédéterminé, le bloc de commande électronique enclanche périodiquement le moteur électrique 11. La rotation de l'arbre du moteur 11 est transmise par l'intermédiaire du réducteur 12 jusqu'à la came 13 qui transmet au fléau 51 le mouvement de roulement alternatif qui est ensuite transmis à la bague 46. Par le biais des tirants rigides 49 le roulement alternatif qui est transmis aux autres bagues 42 qui sont reliées successivement ; puis, par le biais des cliquets 43, un roulement alternatif qui est transmis aux roues à crochet 41. Les tiges filetées d'extension 31a, 31b, 31c se dévissent des écrous 33a, 33b, 33c, ainsi que les tiges filetées d'extension 32a, 32b, 32c des écrous 34a, 34b, 34c entraînant le déplacement des appuis 25 l'un par rapport à l'autre, le châssis restant immobile, et donc le déplacement des fragments osseux fixés dans les appuis 25 par rapport au fragment médian qui est fixé par les broches qui sont elles-mêmes fixées sur ledit châssis.

La variante d'exécution de l'appareil pour ostéosynthèse à extension automatique selon l'invention dans laquelle les tiges filetées d'extension 31a, 31b, 31c ont un filet dont le pas n'est pas égal à celui du filet des tiges filetées d'extension 31a, 32b, 32c, fonctionne de façon analogue à la variante précédente. La grandeur de la discontinuité de déplacement des centres d'appui 25 change en fonction de la valeur de la somme des pas choisis.

La variante de réalisation de l'appareil selon l'invention dans laquelle le châssis ne porte pas les broches de fixation est analogue à la précédente, à cela près que les filets des tiges filetées de d'extension 31a, 31b, 31c ont par rapport aux tiges filetées de d'extension 32a, 32b, 32c le même sens mais un pas différent. Le fonctionnement de cette variante est identique à celui de la variante précédente, elle fonctionne de la même façon. Les appuis 25 effectuent un déplacement relatif dont la discontinuité dépend de la valeur de la différence des pas sélectionnés.

Application industrielle de l'invention

L'appareil à extension automatique pour ostéosynthèse selon l'invention permet, à un régime automatiquement régulé de fonctionnement, d'allonger les membres aussi bien dans un seul que dans deux niveaux, avec un déplacement journalier différent de celui du fragment osseux, ce qui assure la diminution du délai de traitement des patients. Tout ceci permet de placer le patient dans des conditions psycho-émotives favorables et ainsi d'obtenir une forte activité du processus de guérison.

A la différence des appareils d'extension connus sans commande du régime de déplacement automatique, l'appareil à extension automatique pour ostéosynthèse selon l'invention permet d'obtenir les régimes optimaux d'extension en fonction d'un programme donné, au cours duquel il n'y a pas de traumatisme des tissus biologiques et de troubles fonctionnels irréversibles, et le processus de croissance des tissus se rapproche grâce à l'extension du processus de croissance physiologique normale de ces tissus.

L'appareil selon l'invention peut être utilisé dans les travaux de recherche pour l'étude des régularités de la régénération réparative.

L'appareil à extension automatique pour ostéosynthèse selon l'invention est de faible encombrement, de petite masse et peut être stérilisé. De plus son fonctionnement est fiable et silencieux.

Revendications

1. Appareil à extension automatique pour ostéosynthèse, du type comportant deux centres d'appui (1, 24) qui comprennent un appui (2, 25) et des broches de fixation (3) y sont fixées, ainsi qu'un actionneur (5, 39) cinématiquement relié à des mécanismes par crochet et cliquet (7, 36, 37, 38) qui comprennent une roue à crochet (17, 41) et un cliquet à ressort (18, 43), caractérisé en ce que l'actionneur (5, 39) et les mécanismes par crochet et cliquet (7, 36, 37, 38) sont montés sur un châssis (4, 26) qui est placé entre les centres d'appui (1, 24) et y est relié, au moins l'un des centres d'appui (1, 24) est relié au châssis (4, 26), avec possibilité de déplacement relatif au moyen de tiges filetées d'extension qui sont elles-mêmes (8, 31a, 31b, 31c, 32a, 32b, 32c) rigidement et coaxialement reliées aux roues à crochet (17, 41) et constituent un système vis et écrou avec des écrous (9, 33a, 33b, 33c, 34a, 34b, 34c) installés sur le centre d'appui correspondant (1, 24).

2. Appareil selon la revendication 1, caractérisé en ce que l'actionneur (5, 39) des mécanismes par crochet et cliquet comprend un moteur électrique (11) avec un réducteur (12) sur l'arbre duquel est rigidement fixé une came (13) qui coopère avec un mécanisme (6, 50) de la conversion du mouvement rotatif en mouvement alternatif qui est lui-même solidaire de cliquets à ressort (18, 43) desdits mécanismes par crochet et cliquet (7, 36, 37, 38) placés sur des bagues (20, 42, 46) et installées, avec possibilité de rotation sur un axe (21, 45) rigidement fixé sur le châssis (4, 26), la bague (20, 42, 46) étant installée coaxialement à la roue à crochet (17, 41), avec possibilité de rotation par rapport à cette dernière.

3. Appareil selon la revendication 2, caractérisé en ce que le châssis (4, 26) a un rebord extérieur fermé et le mécanisme (6, 50) de conversion du mouvement rotatif en mouvement alternatif a la forme d'un palier (15) et possède un boîtier (14), installé sur la came (13), ledit boîtier (14) étant successivement relié par le biais d'un tirant (22, 49) aux bagues (20, 42, 46).

4. Appareil selon la revendication 2, caractérisé en ce que le châssis (26) à un rebord extérieur ouvert, et le mécanisme (50) de conversion du mouvement rotatif en mouvement alternatif a la forme d'un fléau dont l'un des embouts comporte une fourche (53) entourant la came (13), et l'autre est disposé dans une rainure (47) exécutée à la périphérie de l'une des bagues (42, 46), la bague portant la rainure (46) étant connectée successivement avec les autres bagues (42) par le biais de tirants (49) en forme de fixation articulée.

5. Appareil selon la revendication 2, caractérisé en ce que le châssis (4) est rigidement raccordé à l'un des centres d'appui (1) et les tiges filetées d'extension (8) sont situées de l'autre côté du plan du châssis (4), la roue à crochet (17) est installée, avec possibilité de rotation, sur l'axe susmentionné (21) qui est lui-même rigidement fixé sur le châssis (4).

6. Appareil selon la revendication 2, caractérisé en ce que les axes (45) sont de forme creuse, et les tiges filetées d'extension (31a, 31b, 31c, 32a, 32b, 32c) sont disposées sur les différents côtés des roues à crochet (41), les tiges filetées d'extension (32a, 32b, 32c) étant disposées sur l'un des côtés des roues à crochet (41) et installées, avec possibilité de rotation à l'intérieur des axes creux 45.

7. Appareil selon la revendication 6, caractérisé en ce que les tiges filetées d'extension (31a, 31b, 31c, 32a, 32b, 32c) qui sont disposées sur les différents côtés des roues à crochet (41) sont munies de filets de sens différents.

8. Appareil selon la revendication 7, caractérisé en ce que les filets des tiges d'extension (31a, 31b, 31c, 32a, 32b, 32c) qui sont disposés sur les différents côtés des roues à crochet (41) ont un pas différent les unes des autres.

9. Appareil selon la revendication 6, caractérisé en ce que les tiges filetées d'extension (31a, 31b, 31c, 32a, 32b, 32c) situées de part et d'autre des roues à crochet (41) ont des filets de sens identique mais de pas différent.

10. Appareil selon la revendication 6, caractérisé en ce que le châssis (26) comporte des broches de fixation (3).

11. Appareil selon la revendication 2, caractérisé en ce que l'écrou (34a, 34b, 34c) du système vis et écrou est en forme de tube dont l'un des embouts est relié au centre d'appui (24), alors que la tige filetée d'extension (32a, 32b, 32c) coopère avec le filetage interne exécuté à l'autre embout de ce tube (34a, 34b, 34c).

12. Appareil selon la revendication 11, caractérisé en ce que le tube (34a, 34b, 34c) et le centre d'appui (24) sont raccordés par l'intermédiaire d'une articulation à rotule (35).

13. Appareil selon la revendication 12, caractérisé en ce que les éléments de l'articulation à rotule (35) sont reliés les uns aux autres par un ressort (56).

14. Appareil selon l'une des revendications 1 à 13 caractérisé en ce que les roues à crochet (41) sont munies de dents de sens différents, et que les filets des tiges d'extension (31a, 31b, 31c, 32a, 32b, 32c) qui sont rigidement liés aux roues à crochet (41) de sens correspondant.

FIG.1

FIG.2

FIG.3

FIG.4

FIG.5

FIG.6

FIG.7

# INTERNATIONAL SEARCH REPORT

International Application No PCT/SU 88/00132

## I. CLASSIFICATION OF SUBJECT MATTER (if several classification symbols apply, indicate all) *

According to International Patent Classification (IPC) or to both National Classification and IPC

IPC$^4$  A61B 17/60

## II. FIELDS SEARCHED

### Minimum Documentation Searched [7]

| Classification System | Classification Symbols |
|---|---|
| IPC$^4$ | A61B 17/56, 17/58, 17/60 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | SU, A1, 1122306 (B.V. Shilov) 7 November 1984 (07.11.84) see the claims, figure 4 (cited in the description) | 2 |
| A | SU, A1, 1122308 (Kurgansky nauchno-issledovatelsky institut experimental-noi i klinicheskoi ortopedii i travma-tologii) 7 November 1984 (07.11.84) see the claims, figures 1,2 (cited in the description) | 1,2 |
| A | US, A, 4745913 (ENRICO CASTAMAN and others) 24 May 1988 (24.05.88) see the claims, figure 1 | 1 |

_____

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 22 February 1989 (22.02.89) | 3 April 1989 (03.04.89) |

| International Searching Authority | Signature of Authorized Officer |
|---|---|
| ISA/SU | |

Form PCT/ISA/210 (second sheet) (January 1985)